# EUROPEAN PATENT SPECIFICATION

(11) **EP 0 526 524 B1**
(45) Date of publication and mention of the grant of the patent: **13.12.1995**
(21) Application number: 91908384.0
(22) Date of filing: 24.04.1991
(51) Int. Cl.: A61K 9/20, A61K 9/70

(54) **PHARMACEUTICAL COMPOSITIONS**
ARZNEIMITTEL
COMPOSITIONS PHARMACEUTIQUES

(30) Priority: 26.04.1990 GB 9009390
(43) Date of publication of application: 10.02.1993
(73) Proprietor: SMITH KLINE & FRENCH LABORATORIES LIMITED, Brentford, Middlesex TW8 9EP (GB)
(72) Inventor: TOVEY, Geoffrey, David SmithKline Beecham, Hertfordshire AL7 1EY (GB)
(74) Representative: Florence, Julia Anne
(86) International application number: GB9100651
(87) International publication number: WO9116041

(56) References cited:
- DE-C- 871 821
- FR-A- 2 514 642
- FR-A- 2 571 253
- GB-A- 2 022 999
- GB-A- 2 085 299

## Description

The present invention relates to a pharmaceutical composition for the delivery of medicaments which are absorbed through the sub-lingual mucosa, and to a method for the preparation of such a composition.

It is known to administer medicaments to the oral mucosa, for example the sub-lingual mucosa. Administration by this route has been used when, as in the case of nitroglycerine and nifedipine, it is desired to cause the medicament to be transported into the bloodstream rapidly in order to achieve a rapid therapeutic effect. Another reason for administering a medicament for absorption by the sub-lingual mucosa, rather than by the intestinal mucosa as is the case with most oral dosage forms, would be the instability of certain medicaments, e.g. peptides or hormones, to the physiological environment in the stomach and intestines. Various types of composition have been disclosed in the art as being suitable for the administration of medicaments to the oral mucosa. Such compositions include aerosol sprays (e.g. Nitrolingual™), soft gelatin capsules which can be ruptured and then placed under the tongue to release their liquid contents (e.g. Adalat™ soft gelatine capsules), sub-lingual tablets, and various sub-lingual and buccal patches and membranes containing medicaments.

Certain disadvantages are inherent in many of the prior art compositions. Thus, for example, the application to the sub-lingual mucosa of a spray or liquid formulation can be an inefficient means of administering a medicament since much of the liquid will drain away down the throat. Sub-lingual tablets can suffer from the disadvantage of being difficult to retain under the tongue for a time sufficient to permit adequate absorption of the drug and certain of the buccal patch and membrane dosage forms suffer from the disadvantage of technical complexity and, moreover, are not edible, that is to say they are not degraded readily in the body and must be removed from the mouth for disposal.

UK Patent Application 2022999A describes a vehicle for the oral administration of a substance or composition, said vehicle comprising edible paper. No specific substances or compositions are described therein. UK Patent Application 2166348A describes a sheet-like preparation for administration to the oral cavity, which comprises a drug, gelatin or agar, gluten, a carboxyvinylpolymer, a polyhydric alcohol, a gum and wax, as its essential constituents.

There has now been discovered a composition which offers the advantage of cheapness and simplicity of manufacture; which is edible and therefore does not give rise to disposal problems; which is able to conform to the shape of, and adhere to, the mucosa when hydrated by saliva thereby allowing efficient localised delivery of the medicament; and which permits a steady leaching out of the medicament at a rate which allows its efficient absorption across the mucosal membrane, rather than giving rise to a bolus dose much of which is subsequently washed down the throat by salivary juices; and which is shaped to fit more comfortably against the mucosal site of delivery.

In a first aspect, the present invention provides a pharmaceutical composition for the delivery of medicaments which are absorbed through the sub-lingual mucosa, which composition comprises one or more such medicaments selected from prostaglandins, vaccines, peptides such as a growth hormone e.g. human growth hormone, or a calcitonin, nicotine, nifedipine, auranofin, fenoldopam and glyceryl trinitrate; and a solid carrier which is a wafer formed substantially from starch, the wafer being of a thickness which permits it to be moulded to the contours of the sub-lingual cavity following hydration with saliva thereby allowing localised delivery of the medicament.

Suitably the wafer has a thickness of 0.3 to 1.0 mm, preferably 0.5 to 0.9 mm.

The dimensions of the wafer must be such that it can be easily accommodated in the sub-lingual cavity. Suitably the largest dimension of the wafer is between 10 and 40 mm.

For delivery of a medicament to the sub-lingual mucosa a wafer is suitably placed under the tongue whereupon it softens and moulds to the shape of the mucosal surface with which it is in contact. In this position it adheres reasonably well for sufficient time to allow release of medicament to the sub-lingual mucosa. The wafer can be self-administered or administered by a third party particularly in the case of semi- or unconscious patients.

Preferably the wafer is shaped to provide a cut-away region to accommodate the frenum. In particular the wafer is substantially crescent-moon-shaped. Suitable dimensions for such a shape are an overall length of 10 to 40 mm and an overall width of 5-15 mm, particularly 19 mm by 10.5 mm.

Suitably the wafer comprises any pharmaceutically acceptable starch such as maize, wheat, potato, rice or soya starch or mixtures thereof together with water and optionally a lubricant or emulsifier such as soya starch or a suitable vegetable oil such as rape seed oil. If desired the starch may be pregelatinised. Preferably the wafer is formed from rice paper which may be obtained from G.T. Culpitt & Son Ltd., Wheathampstead, Herts., England.

Such rice papers suitably comprise:

| | %w/w |
|---|---|
| Water | 5.0 - 20.0 |
| Starch | 80.0 - 95.0 |
| Lubricant | 0 - 0.5 |
| Emulsifier | 0 - 0.5 |

The pharmaceutical compositions of the present invention comprise a medicament which can be absorbed through the sub-lingual mucosa, in particular a medicament which is unstable in the physiological environment of the stomach or intestines or which has diminished oral bioavailability due to first past metabolism via the liver, said medicaments being selected from prostaglandins, vaccines, peptides such as a growth hormone e.g. human growth hormone, or a calcitonin, nicotine, nifedipine, auranofin, fenoldopam and glyceryl trinitrate. A unit dose suitably comprises no more than 50 mg of medicament preferably no more than 25 mg.

By the term 'a calcitonin' is meant both naturally occurring calcitonins or derivatives and analogues thereof. Examples of naturally occurring calcitonins include human calcitonin (CAS RN : 21215-62-3), rat calcitonin (CAS RN : 11118-25-5), salmon calcitonin (CAS RN : 47931-85-1, eel calcitonin (CAS RN : 57-14-02-5), reduced chicken calcitonin I (CAS RN : 96157-98-1), chicken calcitonin II (CAS RN : 103468-65-1), ox calcitonin (CAS RN : 26112-29-8), pig calcitonin (CAS RN : 12321-44-7) or sheep calcitonin (CAS RN : 40988-57-6).

Examples of synthetic calcitonins include the des-[Ser², Tyr²²]-Gly⁸-calcitonins described in US-A-4,597,900, the des-[Tyr²²]-salmon calcitonins described in US-A-4,304,692, and the 1,7-dicarbacalcitonins such as eel 1,7-dicarbacalcitonin (elcatonin CAS RN : 60731-46-6), salmon 1,7-dicarbacalcitonin (CAS RN : 60864-37-1) and human 1,7-dicarbacalcitonin (CAS RN : 66811-56-1).

Preferred medicaments for use in the compositions of the present invention include a calcitonin (in particular eel calcitonin or elcatonin), human growth hormone or nifedipine.

Suitably a wafer comprises a therapeutically effective dose of medicament, e.g. 40-200 international units of a calcitonin, 0.25 to 5 units of human growth hormone, 0.5-10 mg of nifedipine, 0.1 to 4 mg of nicotine or 1-6 mg of auranofin. Alternatively, a wafer comprises a suitable fraction of a therapeutically effective dose of medicament, necessitating the sequential administration of an appropriate number of wafers to provide the desired dose.

The compositions may comprise other excipients such as flavouring agents, absorption enhancers, e.g. a glycyrrhizinate such as ammonium glycyrrhizinate or stability enhancing excipients e.g. protease inhibitors or mixtures thereof.

In one embodiment a hydrogenated oil or fat is applied to one side of a wafer to render that side hydrophobic. Alternatively this could be achieved by chemical treatment, such as silylation. This has the effect of keeping the medicament within the wafer when it is placed in the mouth with the other side of the wafer adjacent to the sub-lingual mucosa allowing the medicament to be adsorbed therefrom. Preferably the wafer would be marked so that a patient would know which side should be placed in contact with the underside of the tongue.

In a further aspect the present invention provides a process for preparing a pharmaceutical composition as hereinbefore defined which comprises bringing into association one or more medicaments with the carrier.

This can be achieved by the following general methods:-
(1) The medicament can be incorporated within the wafer mix prior to forming the wafer. Suitably an aqueous slurry of the wafer ingredients is prepared in a stainless steel tank using a homogeniser. A known portion of the slurry is then injected onto the base plate of a flat press. Both base and top plates are heated to a temperature of about 170-200°C. The top plate is brought down and the slurry is cooked for a suitable time, e.g. 20 seconds. The cooked wafer sheet is removed by vacuum suction and transferred to a curing oven. A suitable combination of temperature and controlled humidity (e.g. 25°C and 90% Relative Humidity) are used in the oven to cure the wafer sheet, typically overnight. The cured sheet can then be cut into the desired wafer dosage form shapes using a suitable cutting device. The addition of medicaments to the mix would be at a level which would give the correct total dose in the area of wafer cut from the final sheet as the sub-lingual dosage form.
(2) The wafer without medicament can be formed as a sheet (or continuous roll) and then passed through a screen printer or other printer type of arrangement (e.g. as described in US-A-4322449) to apply the medicament as a layer on and/or partly absorbed into, the wafer. The wafer sheet may be simultaneously warmed so as to drive off the solution carrier which suitably is water, alcohol or chloroform. The final dosage form is then cut from the dried wafer. The coat is applied at a concentration appropriate for final dose relative to area of the wafer.
(3) As 2 above but the medicament is applied in the form of a spray in a suitable volatile solvent such as water or chloroform.
(4) To the basic sheet (or roll) of wafer without medicament is applied a solution of the medicament by droplet addition or spraying onto a small area contained within the boundary of each of the final wafer dosage units. The individual dosage units can then be cut from the sheet in the usual manner. This process minimises drug losses and is suitable for expensive compounds such as peptides.
(5) The active wafer may be prepared by immersion of a wafer in a medicament solution, the wet wafer being then placed and thereby 'wet sealed' onto a larger wafer section formed to suit the sub-lingual cavity. Size and weight of the active wafer and the solution medicament concentration are such that the required dose is prepared.

In these methods the medicament can be applied uniformly or, where appropriate, concentrated near the inner edge of a crescent-moon-shaped wafer which, when administered, is then close to the base of the tongue.

If desired the medicament could be applied to the wafer with a dye.

The following Examples serve to illustrate the present invention.

### EXAMPLE 1

Preformed rice paper wafers supplied by G.T. Culpitt & Son Ltd. and having the following composition (85.3% maize starch, 4.5% pregelatinised wheat starch, 0.2% vegetable oil and water 10%) were impregnated with nifedipine by a metered-dose solvent application method as outlined below. The method also permits the inclusion of dispersion adjuvants such as Polyethylene Glycols (PEG).

### (a) Minimisation of Nifedipine Exposure to Light

Nifedipine is light sensitive, especially in solution. Minimise exposure to light by carrying out all operations under the minimum level of diffuse reflected yellow light from a source of the appropriate wavelength characteristics.

### (b) Preparation of Nifedipine Solution

Dissolve the nifedipine in chloroform, to give a 2.5% w/v solution, by gentle stirring over two or three minutes. If required the PEG can then be dissolved in the nifedipine solution. (15% w/v PEG 3400 can be used to prepare nifedipine/PEG wafers.) The solutions are made up to final volume with chloroform.

### (c) Wafer Impregnation

Precut wafer sections substantially crescent-moon-shaped (19 mm x 10.5 mm x 0.8 mm) are held by tweezers. Using a validated micropipetting device 100 microlitres of nifedipine solution is applied as evenly as possible to the surface of a wafer. The impregnated wafer is then dried in air until no discernible odour of chloroform remains. A representative number of wafers from each batch are assayed for nifedipine content and residual solvent levels using high performance liquid chromatography.

### EXAMPLE 2

### Calcitonin Wafer

A solution of elcatonin (160 i. units) in 25 microlitres of water was applied to the surface of a precut wafer section (of the type described in Example 1) with an adjustable micropipette set at 25 microlitres. The wafer was allowed to dry under ambient conditions. Wafers so prepared are packed into aluminium foil or blister packs ready for subsequent use.

## Claims (Claims for the following Contracting State(s): AT, BE, CH, LI, DE, DK, FR, GB, IT, LU, NL, SE)

1. A pharmaceutical composition for the delivery of medicaments which are absorbed through the sub-lingual mucosa, which composition comprises one or more such medicaments selected from a prostaglandin, a vaccine, a peptide, nicotine, nifedipine, auranofin, fenoldopam and glyceryl trinitrate, and a solid carrier which is a wafer formed substantially from starch, the wafer being of a thickness which permits it to be moulded to the contours of the sub-lingual cavity following hydration with saliva thereby allowing localised delivery of the medicament.

2. A composition according to claim 1 wherein the medicament is a peptide selected from a growth hormone or a calcitonin.

3. A composition according to claim 2 wherein the growth hormone is human growth hormone.

4. A composition according to any one of claims 1 to 3 wherein the medicament is a calcitonin, human growth hormone or nifedipine.

5. A composition according to claim 2 wherein the calcitonin is selected from : human calcitonin, rat calcitonin, salmon calcitonin, eel calcitonin, reduced chicken calcitonin I, chicken calcitonin II, ox calcitonin, pig calcitonin or sheep calcitonin.

6. A composition according to claim 2 wherein the calcitonin is selected from a des-[Ser², Tyr²²]-Gly⁸-calcitonin des-[Tyr²²]-salmon calcitonin or a 1,7-dicarbacalcitonin.

7. A composition according to claim 5 wherein the 1,7-dicarbacalcitonin is eel 1,7-dicarbacalcitonin (elcatonin).

8. A composition according to any of claims 1 to 7 wherein the wafer has a thickness between 0.3 and 1.0 mm.

9. A composition according to any of claims 1 to 8 wherein the largest dimension of the wafer is between 10 mm and 40 mm.

10. A composition according to any one of claims 1 to 9 wherein the wafer is shaped to provide a cut-away region to accommodate the frenum.

11. A composition according to claim 10 wherein the wafer is substantially crescent-moon-shaped.

12. A composition according to any one of claims 1 to 11 wherein the wafer is formed from any pharmaceutically acceptable starch, water and optionally a lubricant or emulsifier.

13. A composition according to any one of claims 1 to 12 wherein the wafer is formed from rice paper.

14. A composition according to any one of claims 1 to 13 further comprising a flavouring agent, an absorption enhancer, or a stability enhancing excipient or mixtures thereof.

15. A composition according to any one of claims 1 to 14 wherein a hydrogenated oil or fat is applied to one side of the wafer to render that side hydrophobic.

16. A process for preparing a pharmaceutical composition according to claim 1 which comprises bringing one or more such medicaments into association with the carrier.

## Claims (Claims for the following Contracting State(s): ES, GR)

1. A process for preparing a pharmaceutical composition for the delivery of medicaments which are absorbed through the sub-lingual mucosa, which composition comprises one or more such medicaments selected from a prostaglandin, a vaccine, a peptide, nicotine, nifedipine, auranofin, fenoldopam and glyceryl trinitrate, and a solid carrier which is a wafer formed substantially from starch, the wafer being of a thickness which permits it to be moulded to the contours of the sub-lingual cavity following hydration with saliva thereby allowing localised delivery of the medicament, which process comprises bringing one or more such medicaments into association with the carrier.

2. A process according to claim 1 wherein the medicament is a peptide selected from a growth hormone or a calcitonin.

3. A process according to claim 2 wherein the growth hormone is human growth hormone.

4. A process according to any one of claims 1 to 3 wherein the medicament is a calcitonin, human growth hormone or nifedipine.

5. A process according to claim 2 wherein the calcitonin is selected from : human calcitonin, rat calcitonin, salmon calcitonin, eel calcitonin, reduced chicken calcitonin I, chicken calcitonin II, ox calcitonin, pig calcitonin or sheep calcitonin.

6. A process according to claim 2 wherein the calcitonin is selected from a des-[Ser², Tyr²²]-Gly⁸-calcitonin des-[Tyr²²]-salmon calcitonin or a 1,7-dicarbacalcitonin.

7. A process according to claim 5 wherein the 1,7-dicarbacalcitonin is eel 1,7-dicarbacalcitonin (elcatonin).

8. A process according to any of claims 1 to 7 wherein the wafer has a thickness between 0.3 and 1.0 mm.

9. A process according to any of claims 1 to 8 wherein the largest dimension of the wafer is between 10 mm and 40 mm.

10. A process according to any one of claims 1 to 9 wherein the wafer is shaped to provide a cut-away region to accommodate the frenum.

11. A process according to claim 10 wherein the wafer is substantially crescent-moon-shaped.

12. A process according to any one of claims 1 to 11 wherein the wafer is formed from any pharmaceutically acceptable starch, water and optionally a lubricant or emulsifier.

13. A process according to any one of claims 1 to 12 wherein the wafer is formed from rice paper.

14. A process according to any one of claims 1 to 13 further comprising a flavouring agent, an absorption enhancer, or a stability enhancing excipient or mixtures thereof.

15. A process according to any one of claims 1 to 14 wherein a hydrogenated oil or fat is applied to one side of the wafer to render that side hydrophobic.

16. A process for preparing a pharmaceutical composition according to any of claims 1 to 15 wherein the medicament is incorporated within the wafer mix prior to forming the wafer.

17. A process according to claim 16 wherein an aqueous slurry of the wafer ingredients is cooked in the form of a sheet, the cooked wafer sheet cured and cut into the desired wafer dosage form shapes.

18. A process according to any of claims 1 to 15 wherein the wafer without medicament is formed as a sheet or continuous roll, passed through a printer to apply the medicament as a layer on and/or partly adsorbed into, the wafer,and the final dosage form cut from the dried wafer.

19. A process according to any of claims 1 to 15 wherein the wafer without medicament is formed as a sheet or continuous roll, the medicament is applied in the form of a spray in a volatile solvent and the final dosage form cut from the dried wafer.

20. A process according to any of claims 1 to 15 wherein the wafer without medicament is formed as a sheet or continuous roll, a solution of the medicament is applied by droplet addition or spraying onto a small area contained within the boundary of each of the final dosage units and the final dosage form cut from the dried wafer.

21. A process according to any of claims 1 to 15 wherein the wafer is immersed in a medicament solution and the wet wafer is then placed and thereby 'wet sealed' onto a larger wafer section formed to suit the sublingual cavity.

## Patentansprüche (Patentansprüche für folgende(n) Vertragsstaat(en): AT, BE, CH, LI, DE, DK, FR, GB, IT, LU, NL, SE)

1. Arzneimittel zur Abgabe Von Arzneistoffen, die über die sublinguale Schleimhaut absorbiert werden, wobei das Mittel ein oder mehrere Arzneistoffe, ausgewählt aus einem Prostaglandin, einem Impfstoff, einem Peptid, Nikotin, Nifedipin, Auranofin, Fenoldopam und Glycerintrinitrat, und einen festen Träger umfaßt, der eine im wesentlichen aus Stärke gebildete Oblate ist, wobei die Oblate eine Dicke aufweist, die gestattet, daß sie nach der Hydratation mit Speichel zu den Konturen der sublingualen Höhle geformt wird, wodurch eine lokalisierte Abgabe des Arzneistoffs ermöglicht wird.

2. Mittel nach Anspruch 1, wobei der Arzneistoff ein Peptid ist, das aus einem Wachstumshormon oder einem Calcitonin ausgewählt wird .

3. Mittel nach Anspruch 2, wobei das Wachstumshormon menschliches Wachstumshormon ist.

4. Mittel nach einem der Ansprüche 1 bis 3, wobei der Arzneistoff ein Calcitonin, menschliches Wachstumshormon oder Nifedipin ist.

5. Mittel nach Anspruch 2, wobei das Calcitonin aus menschlichem Calcitonin, Rattencalcitonin, Lachscalcitonin, Aalcalcitonin, reduziertem Hühnercalcitonin I, Hühnercalcitonin II, Ochsencalcitonin, Schweinecalcitonin oder Schafcalcitonin ausgewählt wird .

6. Mittel nach Anspruch 2, wobei das Calcitonin aus einem Des-[Ser²,Tyr²²]-Gly⁸-calcitonin, Des-[Tyr²²]-Lachscalcitonin oder einem 1,7-Dicarbacalcitonin ausgewählt wird.

7. Mittel nach Anspruch 5, wobei das 1,7-Dicarbacalcitonin Aal-1,7-dicarbacalcitonin (Elcatonin) ist.

8. Mittel nach einem der Ansprüche 1 bis 7, wobei die Oblate eine Dicke zwischen 0,3 und 1,0 mm aufweist.

9. Mittel nach einem der Ansprüche 1 bis 8, wobei die größte Abmessung der Oblate zwischen 10 mm und 40 mm liegt.

10. Mittel nach einem der Ansprüche 1 bis 9, wobei die Oblate so geformt ist, daß ein Ausschnittsbereich zur Aufnahme des Frenulums bereitgestellt wird.

11. Mittel nach Anspruch 10, wobei die Oblate im wesentlichen halbmondförmig ist.

12. Mittel nach einem der Ansprüche 1 bis 11, wobei die Oblate aus einer beliebigen pharmazeutisch verträglichen Stärke, Wasser und gegebenenfalls einem Gleitmittel oder Emulgator gebildet wird.

13. Mittel nach einem der Ansprüche 1 bis 12, wobei die Oblate aus Reispapier gebildet wird.

14. Mittel nach einem der Ansprüche 1 bis 13, das weiter einen Aromastoff, einen Absorptionsverstärker oder einen stabilitätserhöhenden Arzneimittelträger oder Gemische daraus umfaßt.

15. Mittel nach einem der Ansprüche 1 bis 14, wobei auf eine Seite der Oblate ein gehärtetes Öl oder Fett aufgetragen ist, um diese Seite hydrophob zu machen .

16. Verfahren zur Herstellung eines Arzneimittels nach Anspruch 1, das das Zusammenbringen eines oder mehrerer der Arzneistoffe mit dem Träger umfaßt.

## Patentansprüche (Patentansprüche für folgende(n) Vertragsstaat(en): ES, GR)

1. Verfahren zur Herstellung eines Arzneimittels zur Abgabe von Arzneistoffen, die durch die sublinguale Schleimhaut absorbiert werden, wobei das Mittel ein oder mehrere Arzneistoffe ausgewählt aus einem Prostaglandin, einem Impfstoff, einem Peptid, Nikotin, Nifedipin, Auranofin, Fenoldopam und Glycerintrinitrat, und einen festen Träger umfaßt, der eine im wesentlichen aus Stärke gebildete Oblate ist, wobei die Oblate eine Dicke aufweist, die gestattet, daß sie nach der Hydratation mit Speichel zu den Konturen der sublingualen Höhle geformt wird, wodurch eine lokalisierte Abgabe des Arzneistoffs erlaubt wird, wobei das Verfahren das Zusammenbringen eines oder mehrerer solcher Arzneistoffe mit dem Träger umfaßt.

2. Verfahren nach Anspruch 1, wobei der Arzneistoff ein Peptid ist, das aus einem Wachstumshormon oder einem Calcitonin ausgewählt wird.

3. Verfahren nach Anspruch 2, wobei das Wachstumshormon menschliches Wachstumshormon ist.

4. Verfahren nach einem der Ansprüche 1 bis 3, wobei der Arzneistoff ein Calcitonin, menschliches Wachstumshormon oder Nifedipin ist.

5. Verfahren nach Anspruch 2, wobei das Calcitonin aus menschlichem Calcitonin, Rattencalcitonin, Lachscalcitonin, Aalcalcitonin, reduziertem Hühnercalcitonin I, Hühnercalcitonin II, Ochsencalcitonin, Schweinecalcitonin oder Schafcalcitonin ausgewählt wird.

6. Verfahren nach Anspruch 2, wobei das Calcitonin aus einem Des-[Ser²,Tyr²²]-Gly⁸-calcitonin, Des-[Tyr²²]-Lachscalcitonin oder einem 1,7-Dicarbacalcitonin ausgewählt wird.

7. Verfahren nach Anspruch 5, wobei das 1,7-Dicarbacalcitonin Aal-1,7-dicarbacalcitonin (Elcatonin) ist.

8. Verfahren nach einem der Ansprüche 1 bis 7, wobei die Oblate eine Dicke zwischen 0,3 und 1,0 mm aufweist.

9. Verfahren nach einem der Ansprüche 1 bis 8, wobei die größte Abmessung der Oblate zwischen 10 mm und 40 mm liegt.

10. Verfahren nach einem der Ansprüche 1 bis 9, wobei die Oblate so geformt ist, daß ein Ausschnittsbereich zur Aufnahme des Frenulums bereitgestellt wird.

11. Verfahren nach Anspruch 10, wobei die Oblate im wesentlichen halbmondförmig ist.

12. Verfahren nach einem der Ansprüche 1 bis 11, wobei die Oblate aus einer beliebigen pharmazeutisch verträglichen Stärke, Wasser und gegebenenfalls einem Gleitmittel oder Emulgator gebildet wird.

13. Verfahren nach einem der Ansprüche 1 bis 12, wobei die Oblate aus Reispapier gebildet wird.

14. Verfahren nach einem der Ansprüche 1 bis 13, das weiter einen Aromastoff, einen Absorptionsverstärker oder einen stabilitätserhöhenden Arzneimittelträger oder Gemische daraus umfaßt.

15. Verfahren nach einem der Ansprüche 1 bis 14, wobei auf eine Seite der Oblate ein gehärtetes Öl oder Fett aufgetragen ist, um diese Seite hydrophob zu machen.

16. Verfahren zur Herstellung eines Arzneimittels nach einem der Ansprüche 1 bis 15, wobei der Arzneistoff vor dem Formen der Oblate in das Oblatengemisch eingebracht wird.

17. Verfahren nach Anspruch 16, wobei eine wäßrige Aufschlämmung der Oblatenbestandteile in Form eines Blattes gebacken, das gebackene Oblatenblatt gehärtet und in die gewünschten Formen für die Dosierung der Oblate geschnitten wird.

18. Verfahren nach einem der Ansprüche 1 bis 15, wobei die Oblate ohne Arzneistoff als Blatt oder Endlosrolle geformt wird, durch einen Drucker geleitet wird, wobei der Arzneistoff als Schicht auf die Oblate aufgetragen und/oder teilweise in die Oblate adsorbiert wird, und die Enddosierungsform aus der getrockneten Oblate geschnitten wird.

19. Verfahren nach einem der Ansprüche 1 bis 15, wobei die Oblate ohne Arzneistoff als Blatt oder Endlosrolle geformt, der Arzneistoff als Spray in einem flüchtigen Lösungsmittel aufgetragen und die Enddosierungsform aus der getrockneten Oblate ausgeschnitten wird.

20. Verfahren nach einem der Ansprüche 1 bis 15, wobei die Oblate ohne Arzneistoff als Blatt oder Endlosrolle geformt, eine Lösung des Arzneistoffs durch Tröpfchenzugabe oder Sprühen auf eine kleine Fläche, die innerhalb der Umgrenzung von jeder Enddosierungseinheit enthalten ist, aufgetragen und die Enddosierungsform aus der getrockneten Oblate ausgeschnitten wird.

21. Verfahren nach einem der Ansprüche 1 bis 15, wobei die Oblate in eine Arzneistofflösung eingetaucht und die feuchte Oblate dann auf einen größeren Oblatenabschnitt gelegt und dadurch 'feuchtversiegelt' wird, der so geformt ist, daß er in die sublinguale Höhle paßt.

## Revendications (Revendications pour l'(les) Etat(s) contractant(s) suivant(s): AT, BE, CH, LI, DE, DK, FR, GB, IT, LU, NL, SE)

1. Composition pharmaceutique pour la libération de médicaments qui sont absorbés à travers la muqueuse sublinguale, comprenant un ou plusieurs médicaments de ce type, choisis parmi une prostaglandine, un vaccin, un peptide, la nicotine, la nifédipine, l'auranofine, le fénoldopam et le trinitrate de glycéryle, ainsi qu'un support solide qui est une pastille formée essentiellement d'amidon, ladite pastille ayant une épaisseur qui lui permet de s'adapter aux contours de la cavité sublinguale après hydratation avec la salive, permettant ainsi une libération localisée du médicament.

2. Composition selon la revendication 1, dans laquelle le médicament est un peptide choisi parmi une hormone de croissance ou une calcitonine.

3. Composition selon la revendication 2, dans laquelle l'hormone de croissance est une hormone de croissance humaine.

4. Composition selon l'une quelconque des revendications 1 à 3, dans laquelle le médicament est une calcitonine, une hormone de croissance humaine ou la nifédipine.

5. Composition selon la revendication 2, dans laquelle la calcitonine est choisie parmi la calcitonine humaine, la calcitonine de rat, la calcitonine de saumon, la calcitonine d'anguille, la calcitonine I réduite de poulet, la calcitonine II de poulet, la calcitonine de boeuf, la calcitonine de porc ou la calcitonine de mouton.

6. Composition selon la revendication 2, dans laquelle la calcitonine est choisie parmi une des-[Ser²,Tyr²²]-Gly⁸-calcitonine, une des-[Tyr²²]-calcitonine de saumon ou une 1,7-dicarbacalcitonine.

7. Composition selon la revendication 5, dans laquelle la 1,7-dicarbacalcitonine est la 1,7-dicarbacalcitonine d'anguille (elcatonine).

8. Composition selon l une quelconque des revendications 1 à 7, la pastille ayant une épaisseur comprise entre 0,3 et 1,0 mm.

9. Composition selon l'une quelconque des revendications 1 à 8, la plus grande dimension de la pastille étant comprise entre 10 mm et 40 mm.

10. Composition selon l'une quelconque des revendications 1 à 9, la pastille étant façonnée de façon à fournir une région découpée pour son adaptation au frein de la langue.

11. Composition selon la revendication 10, la pastille ayant essentiellement la forme d'un croissant de lune.

12. Composition selon l'une quelconque des revendications 1 à 11, la pastille étant formée de n'importe quel amidon pharmaceutiquement acceptable, d'eau et éventuellement d'un lubrifiant ou d'un émulsifiant.

13. Composition selon l'une quelconque des revendications 1 à 12, la pastille étant formée de papier de riz.

14. Composition selon l'une quelconque des revendications 1 à 13, comprenant, en outre, un agent aromatisant, un renforceur d'absorption ou un excipient renforçant la stabilité, ou des mélanges de ceux-ci.

15. Composition selon l'une quelconque des revendications 1 à 14, une huile ou une graisse hydrogénée étant appliquée sur une face de la pastille pour rendre cette face hydrophobe.

16. Procédé de préparation d'une composition pharmaceutique selon la revendication 1, comprenant l'association d'un ou plusieurs médicaments avec le support.

## Revendications (Revendications pour l'(les) Etat(s) contractant(s) suivant(s): ES, GR)

1. Procédé de préparation d'une composition pharmaceutique pour la libération de médicaments qui sont absorbés à travers la muqueuse sublinguale, comprenant un ou plusieurs médicaments de ce type, choisis parmi une prostaglandine, un vaccin, un peptide, la nicotine, la nifédipine, l'auranofine, le fénoldopam et le trinitrate de glycéryle, ainsi qu'un support solide qui est une pastille formée essentiellement d'amidon, ladite pastille ayant une épaisseur qui lui permet de s'adapter aux contours de la cavité sublinguale après hydratation avec la salive, permettant ainsi une libération localisée du médicament, ledit procédé comprenant l'association d'un ou plusieurs médicaments de ce type avec le support.

2. Procédé selon la revendication 1, dans lequel le médicament est un peptide choisi parmi une hormone de croissance ou une calcitonine.

3. Procédé selon la revendication 2, dans lequel l'hormone de croissance est une hormone de croissance humaine.

4. Procédé selon l'une quelconque des revendications 1 à 3, dans lequel le médicament est une calcitonine, une hormone de croissance humaine ou la nifédipine.

5. Procédé selon la revendication 2, dans lequel la calcitonine est choisie parmi la calcitonine humaine, la calcitonine de rat, la calcitonine de saumon, la calcitonine d'anguille, la calcitonine I réduite de poulet, la calcitonine II de poulet, la calcitonine de boeuf, la calcitonine de porc ou la calcitonine de mouton.

6. Procédé selon la revendication 2, dans lequel la calcitonine est choisie parmi une des-[Ser²,Tyr²²]-Gly⁸-calcitonine, une des-[Tyr²²]-calcitonine de saumon ou une 1,7-dicarbacalcitonine.

7. Procédé selon la revendication 5, dans lequel la 1,7-dicarbacalcitonine est la 1,7-dicarbacalcitonine d'anguille (elcatonine).

8. Procédé selon l une quelconque des revendications 1 à 7, dans lequel la pastille a une épaisseur comprise entre 0,3 et 1,0 mm.

9. Procédé selon l une quelconque des revendications 1 à 8, la plus grande dimension de la pastille étant comprise entre 10 mm et 40 mm.

10. Procédé selon l'une quelconque des revendications 1 à 9, dans lequel on façonne la pastille de façon à fournir une région découpée pour son adaptation au frein de la langue.

11. Procédé selon la revendication 10, dans lequel la pastille a essentiellement la forme d'un croissant de lune.

12. Procédé selon l'une quelconque des revendications 1 à 11, dans lequel la pastille est formée de n'importe quel amidon pharmaceutiquement acceptable, d'eau et éventuellement d'un lubrifiant ou d'un émulsifiant.

13. Procédé selon l une quelconque des revendications 1 à 12, dans lequel la pastille est formée de papier de riz.

14. Procédé selon l une quelconque des revendications 1 à 13, comprenant, en outre, un agent aromatisant, un renforceur d'absorption ou un excipient renforçant la stabilité, ou des mélanges de ceux-ci.

15. Procédé selon l une quelconque des revendications 1 à 14, dans lequel on applique une huile ou une graisse hydrogénée sur une face de la pastille de façon à rendre cette face hydrophobe.

16. Procédé de préparation d'une composition pharmaceutique selon l une quelconque des revendications 1 à 15, dans lequel on incorpore le médicament dans le mélange constituant la pastille avant de former ladite pastille.

17. Procédé selon la revendication 16, dans lequel on cuit une suspension aqueuse des ingrédients formant la pastille, sous la forme d'une feuille, on durcit la feuille cuite formant la pastille et on la découpe de façon à obtenir les formes unitaires désirées à administrer.

18. Procédé selon l'une quelconque des revendications 1 à 15, dans lequel on façonne la pastille, sans le médicament, en une feuille ou un rouleau continu, on la fait passer dans une presse pour appliquer le médicament sous la forme d'une couche sur la pastille et/ou le faire adsorber partiellement dans celle-ci, et on découpe la dose unitaire finale d'administration dans la pastille séchée.

19. Procédé selon l'une quelconque des revendications 1 à 15, dans lequel on façonne la pastille, sans le médicament, en une feuille ou un rouleau continu, on applique le médicament sous la forme d'un spray dans un solvant volatil et on découpe la dose unitaire finale d'administration dans la pastille séchée.

20. Procédé selon l'une quelconque des revendications 1 à 15, dans lequel on façonne la pastille, sans le médicament, en une feuille ou un rouleau continu, on applique une solution du médicament par addition ou pulvérisation de gouttelettes sur une petite surface contenue dans les limites de chaque unité finale d'administration et on découpe la dose unitaire finale d'administration dans la pastille séchée.

21. Procédé selon l'une quelconque des revendications 1 à 15, dans lequel on immerge la pastille dans une solution de médicament et ensuite, on place la pastille humide sur une section plus grande de pastille, grâce à quoi elle est "scellée par voie humide", ladite pastille étant façonnée de façon à s'adapter aux formes de la cavité sublinguale.
